Europäisches Patentamt

European Patent Office

Office européen des brevets

(19)

(11) Publication number: **0 070 924**
**B1**

(12) **EUROPEAN PATENT SPECIFICATION**

(45) Date of publication of patent specification: **16.04.86**

(21) Application number: **81110104.7**

(22) Date of filing: **03.12.81**

(51) Int. Cl.⁴: **C 07 D 251/64**

(54) **Process for preparing hexacis(methoxymethyl)melamine.**

(30) Priority: **28.07.81 IT 2319381**

(43) Date of publication of application:
**09.02.83 Bulletin 83/06**

(45) Publication of the grant of the patent:
**16.04.86 Bulletin 86/16**

(84) Designated Contracting States:
**AT CH DE FR LI NL**

(56) References cited:
**DE-A-2 716 006**
**DE-A-2 839 713**
**DE-B-1 054 459**
**US-A-2 715 619**
**US-A-2 918 452**

**The file contains technical information
submitted after the application was filed and
not included in this specification**

(73) Proprietor: **CHIMICA POMPONESCO S.p.A.**
**Strada Provinciale Dosolese**
**I-46030 Pomponesco Mantova (IT)**

(72) Inventor: **Albanesi, Giancarlo**
**Politecnico di Milano Piazza Leonardo da Vinci**
**I-20133 Milano (IT)**
Inventor: **Segurini, Gianpaolo**
**c/o Chimica Pomponesco S.p.A. Strada**
**Dosolese**
**I-46030 Pomponesco (Mantova) (IT)**
Inventor: **Mennea, Vittorio**
**c/o Chimica Pomponesco S.p.A. Strada**
**Dosolese**
**I-46030 Pomponesco (Mantova) (IT)**

(74) Representative: **Ferri, Antonio**
**c/o BREVETTI EUROPA S.r.l. Piazza Bernini 6**
**I-20133 Milano (IT)**

EP 0 070 924 B1

## Description

The present invention relates to an improved and simplified process for preparing hexacis-(methoxymethyl)melamine endowed with excellent qualitative characteristics.

Background of the invention

As is known, hexacis(methoxymethyl)-melamine is a product particularly utilized as monomer for the production of resins and lacquers which find a broad use in various appliances, such as for example paints, printing inks, impregnation of paper materials and the like; recently said monomer has been utilized as a substitute for melaminic resins in the melaminic resins/alkyd resins (or -/acrylic resins, or -/epoxy resins) mixed systems. The technically pure hexacis(methoxymethyl)melamine is a solid which melts at about 49°C and possesses an "etherification degree" (average number of methoxy groups per mole) close to the theoretical value, which, as is known, is equal to 6.

The commercial hexacis(methoxymethyl)-melamine has generally an etherification degree lower than 6 and is in the form of a more or less viscous syrup, as it contains more or less considerable amounts of auto-condensation products, which render the above-mentioned product less "selective" in the applicative reactions in respect of the technically pure product.

The preferred chemical form for the hexacis(methoxymethyl)melamine destineted to polycondensate syntheses is the one corresponding to a constant etherification degree and with values higher than 5.8, as for such values the hexacis(methoxymethyl)melamine is in the form of a product characterized by

— a low precondensation, as is required for a correct trend of the mixed polycondensations;
— a liquid physical state, with a low viscosity and corresponding easiness of use in the chemical processes and of handling in the transfer and storing operations;
— a full solubility in water in any ratios, and consequent possibility of use in processes and products involving reactions and utilizations in an aqueous medium.

The preparation of the product in question in the abovesaid preferred form is not easily attainable industrially and it requires in any case operations which involve complex apparatuses and difficult operational modalities, with economical burdens connected therewith. In fact, as is known, hexacis(methoxymethyl)melamine (hereinafter referred to also with the conventional abbreviated symbol $R(OCH_3)_6$—is obtained by etherification, with methanol, of hexacis(hydroxymethyl)melamine (hereinafter referred to also with the conventional abbreviated symbol $R(OH)_6$): such etherification, since it is an equilibrium reaction, must be carried out—for the purposes of efficiency—with sufficiently anhydrous starting products (i.e. $R(OH)_6$ and methanol). Therefore it is necessary, for technical-economical purposes, that the synthesis reaction of $R(OH)_6$ (which precedes said etherification and which is too an equilibrium reaction) may be conducted in an optimum manner both for the sake of the best operativity and profitability of the process, and for the sake of the chemical-physical properties of the desired $R(OH)_6$ intended for being successively etherified to $R(OCH_3)_6$.

In the abovesaid synthesis reaction (carried out by reacting melamine with an excess of formaldehyde in an aqueous solution) $R(OH)_6$ which gradually forms, usually precipitates, in the known processes, in a "get" type form incorporating considerable amounts of water and very difficult to be filtered. By consequence the separation of $R(OH)_6$ (for the necessary recovery of the excess of formaldehyde solution at the conclusion of the synthesis operation) requires complex equipment and operations, in particular:

— the discharge from the synthesis reactor of the whole $R(OH)_6$ produced along with the residual reaction medium (water+ formaldehyde);
— the very difficult separation, by using physical-mechanical means (such as centrifugation, filtration and the like) of $R(OH)_6$ from the residual reaction medium (free and incorporated);
— the reintroduction into said synthesis reactor (or into another reactor) of $R(OH)_6$ so produced and separated, for its etherification with methanol.

The above-mentioned operations for separating $R(OH)_6$ are technically and economically very onerous owing to the chemical-physical state of the abovesaid product, and furthermore they difficultly succeed in yielding a $R(OH)_6$ with the desired dehydration degree.

Summary of the invention

Thus, it is an object of the present invention to provide a process for preparing hexacis(methoxymethyl)melamine by which hexacis(hydroxymethyl)melamine—to be successively etherified to hexacis(methoxymethyl)melamine — is obtained in the form of an easily filterable crystalline powder, said process, by consequence, resulting simplified as regards apparatuses and operative steps, easy to be conducted, and capable of providing high material yields.

Another object of this invention is that of obtaining hexacis(methoxymethyl)melamine with an etherification degree higher than 5.8 and thoroughly soluble in water.

These and still other objects, which will be more clearly apparent to those skilled in the art from the following detailed description and from the examples, are achieved by a process which comprises the synthesis reaction between melamine and a high excess of formaldehyde in aqueous solution for obtaining hexacis(hydroxymethyl)melamine, the separation of the latter by filtration of the residual reaction medium, and its etherification with methanol in order to obtain hexacis(methoxymethyl)melamine, the abovesaid synthesis reaction of hexacis(hydroxymethyl)melamine being carried out, according to the present invention, by starting and conducting said reaction at a pH value not lower than 7.5, by keeping the reaction mass under stirring at a stirring speed sufficient to maintain the hexacis(hydroxymethyl)melamine, precipitated in the reaction medium, in the form of a crystalline powder suspended in the same reaction medium in a substantially homogeneous manner, and by maintaining the temperature of the reaction mass and of the mechanical parts in contact with the latter at a value not below 40°C, and finally by separating by filtration from the residual reaction medium, kept at a temperature not lower than 40°C, said powder of hexacis(hydroxymethyl)melamine so obtained in a crystalline easily filterable form, incorporating a few water and ready for its successive etherification.

Description of the preferred embodiment

A preferred but not exclusive embodiment of the present invention is carried out by effecting the synthesis of $R(OH)_6$ by reacting—in a approximate molar ratio of 1 to 11—melamine and formaldehyde (the latter in the form of an aqueous solution at 44% by weight of formaldehyde) at a pH from 9 to 9.8 (obtained by addition of a NaOH aqueous solution) and at a temperature not lower than 40°C, keeping the reacting mass under a gentle stirring just sufficient to prevent the $R(OH)_6$ powder which forms in said synthesis in the desired crystalline form from decanting on the bottom of the reactor; the latter is of a conventional known type, with the characteristic of being equipped with a bottom discharge complete with a gauze filter. At the end of the abovesaid synthesis reaction, the residual liquid of such synthesis (essentially water and formaldehyde), maintained at a temperature ranging from 40°C to 50°C, is discharged; the powder of $R(OH)_6$, easily retained in the reactor by the above-mentioned gauze filter, since said powder is—as desired—in an easily filterable crystalline form substantially free from incorporated water, being conversely left in the reactor; finally, always operating in the same reactor, etherification of said $R(OH)_6$ with methanol is carried out by employing a conventional technique and by catalysis acidified by HCl, thus obtaining the desired $R(OCH_3)_6$ having an etherification degree higher than 5.8 and being thoroughly soluble in water in any ratio.

The following three examples of embodiments of the present invention are given for illustrative purposes, without being however a limitation of the invention.

Example 1

This example illustrates the process according to this invention, practised by using process equipments known to a technician skilled in the art.

The reactor utilized for the aforesaid synthesis of hexacis(hydroxymethyl)melamine and successively for its etherification to hexacis(methoxymethyl)melamine, was of the conventional vertical type (total volume: 13 m³) equipped with a blade stirrer with motor-driven variable speed gear, with an external half-pipe jacket for the heating (by means of steam) or the cooling (by means of water), with a tube bundle condenser, with the usual devices for the control of the process parameters (pH, temperature, pressure), and with a bottom discharge provided with a gauze filter with square meshes of 0.25 mm side, known in the art.

The apparatuses upstream and downstream of said reactor were all of the conventional type.

Said hexacis(hydroxymethyl)melamine synthesis was effected in the abovesaid reactor by operating—according to the present invention— as follows:

— 3752 kg of an aqueous solution at 44% by weight of formaldehyde were introduced into said reactor, the stirrer was started at 5 rpm, the pH was adjusted at a value of 9.4 by NaOH aqueous solution, 630 kg of melamine were added, whereupon the reaction between melamine and formaldehyde was started raising the temperature to about 70°C: said reaction (which was exothermic) was allowed to proceed for 1 hour with temperature peaks up to 75°C, the reaction being successively completed in a time period of three hours by gradually decreasing the temperature down to 45°C, keeping the pH at a value higher than 9 (about 9.4) and gradually increasing the stirrer speed from 5 to 15 rpm.

At the conclusion of said synthesis, the stirrer was stopped and, while maintaining the temperature at about 45°C and the pH at about 9.4, the formaldehyde aqueous solution remained from said synthesis was discharged from the reactor, while the powder of hexacis-(hydroxymethyl)melamine—so obtained in a crystalline form with particles having dimensions larger than 0.3 mm—remained in said reactor, retained therein by the abovesaid gauze filter, and ready for the immediately successive etherification operation, which took place in the same reactor in the following way and with a known technology:

— 3440 kg of methanol were charged and 160 kg of an aqueous solution at 33% by weight of HCl were added, bringing the pH to a value of 0.7—0.9; the temperature was kept at a value of 40°C and the stirrer speed at 30—40 rpm: etherification was completed after 10 minutes from the complete solubilization of the hexacis(hydroxymethyl)melamine powder.

At the end of the etherification step described hereinabove, the pH was brought to a value of 8—8.5 with a NaOH solution, then the residual reaction medium (essentially water and methanol) and the precipitated sodium chloride were separated, so obtaining about 1970 kg of hexacis(methoxymethyl)melamine at 95% on a dry basis (the balance to 100 being essentially water), corresponding to a quantitative yield of said product of about 96% by mole referred to the starting melamine.

The hexacis(methoxymethyl)melamine obtained as described hereinbefore, subjected to chemical analysis, revealed an average etherification degree higher than approx. 5.8, and appeared in the form of a liquid product having a viscosity of 2400 cps, fully soluble in water in any ratio, by consequence easy to be handled in the operations required for the transfers, storages, and applicative reactions thereof.

The etherification reaction described hereinabove was conducted in a very easily controllable manner and with a very rapid kinetics.

Example 2

2400 kg of $R(OCH_3)_6$ were prepared by employing the same procedures and the same apparatuses of example 1, with the only variation that said synthesis of $R(OH)_6$ was carried out at a pH of 8.5. The obtained process results (in particular quantitative yields) and product results (qualitative characteristics of $R(OH)_6$ and $R(OCH_3)_6$) were substantially identical with those of example 1.

Example 3

This example is given to prove the high reliability of the process object of the present invention for the purposes of producing hexacis-(methoxymethyl)melamine having very constant qualitative characteristics at the desired values.

By using the same apparatuses, procedures and amounts of the same substances of example 1, there were prepared, in succession, three discrete lots of about 2400 kg each of hexacis-(methoxymethyl)melamine, which were marked by No. 1, No. 2 and No. 3. From each of said lots an average product sample was drawn, i.e. in total three average samples which were subjected to chemical analysis, the results recorded on Table 1 (referred to the three above-cited products at 95% on a dry basis) having been obtained.

TABLE 1

| | Lot | | |
|---|---|---|---|
| | No. 1 | No. 2 | No. 3 |
| Methylation degree | 5.8 | 5.9 | 5.8 |
| Viscosity, cps (20°C) (Höppler viscosimeter) | 2300 | 1900 | 2100 |
| Refractive index ($n_\mathrm{ni}^{20}$) | 1.517 | 1.515 | 1.516 |

From what above described and exemplified there are apparent the advantages of the present invention, such advantages being briefly summarizable as follows:

— simplification of the production plant;
— simplification and operative easiness of the process;
— qualitative characteristics of hexacis(methoxymethyl)melamine at an optimum and constant degree, in particular the low tendency to self-cross-linking: as is known, this fact is of essential importance for a product—such as hexacis(methoxymethyl)melamine—which is prevailingly utilized in mixed polymeric systems.

The process object of the present invention as described and illustrated hereinbefore may be carried out with technically equivalent modifications and variations, but all falling within the scope of the inventive concept of the present invention.

**Claims**

1. A process for preparing water-soluble hexacis(methoxymethyl)melamine, comprising the methylolation reaction of melamine with formaldehyde in an aqueous solution for obtaining hexacis(hydroxymethyl)melamine, the separation of the latter from the residual reaction medium and the etherification reaction of said hexacis(hydroxymethyl)melamine with methanol for obtaining hexacis(methoxymethyl)melamine, characterized in that said methylolation reaction comprises:

(a) preparing a mixture of melamine into an aqueous formaldehyde solution having a pH adjusted to about 9, wherein the mol-ratio melamine:formaldehyde is about 1:11, and the water content is of about 44% by weight based on the total weight of said mixture;

(b) reacting under stirring said mixture for about an hour at a temperature of 70° to 75°C, while maintaining the reaction mass at a pH of about 9 under stirring at a stirring speed just sufficient to maintain the hexacis(hydroxymethyl)melamine, which precipitates in the reaction medium, in the form of a crystalline powder substantially homogeneously suspended in such reaction medium;

(c) further reaction under stirring for about 3 hours the reaction mass as obtained in (b) to the completion of the methylolation reaction, lowering and keeping the temperature of said reaction mass and of the mechanical parts in contact therewith at a value of about 45°C, while maintaining the pH at about 9.

(d) separating by filtration, from the residual reaction medium, the so obtained hexacis-(hydroxymethyl)melamine, while maintaining said residual reaction medium at a temperature of about 45°C and at a pH of about 9, said hexacis-(hydroxymethyl)melamine being in the form of a crystalline powder, easily filterable and ready for being successively etherified with methanol to hexacis(methoxymethyl)melamine.

2. The process according to the claim 1, characterized in that the separating by filtration in (d) is accomplished by extracting said residual reaction medium from the reaction-vessel, and said etherification reaction is carried out in the same reaction-vessel.

3. Hexacis(methoxymethyl)melamine prepared by the process according to the claim 1 or 2, characterized in that it is thoroughly soluble in water in any ratio, and in that it has a viscosity of less than 2500 cps (at 20°C and with a solid content of 95%), said hexacis(methoxymethyl)-melamine having a medium methylation degree of not less than 5.8.

**Patentansprüche**

1. Verfahren zur Herstellung von wasserlöslichen Hexakis-(methoxymethyl)-melamin durch Hydroxymethylierung von Melamin mit Formaldehyd in wässriger Lösung zur Herstellung von Hexakis-(hydroxymethyl)-melamin, die Abtrennung des letzteren von dem übrigen Reaktionsmedium und die Veretherung dieses Hexakis-(hydroxymethyl)-melamins mit Methanol zur Herstellung von Hexakis-(methoxymethyl)-melamin, gekennzeichnet dadurch, daß die Hydroxymethylierungsreaktion besteht aus:

(a) Herstellung eines Gemisches aus Melamin und einer wässrigen Formaldehydlösung mit einem pH-Wert, der auf etwa 9 eingestellt ist, worin das Molverhältnis von Melamin:Formaldehyd etwa 1:11 beträgt und der Wassergehalt etwa 44 Gewichts-% des Gesamtgewichtes dieses Gemisches beträgt;

(b) Umsetzung dieses Gemisches bei einer Temperatur von etwa 70 bis 75°C unter Rühren innerhalb einer Stunde, wobei das Reaktionsgemisch bei einem pH-Wert von etwa 9 unter Rühren gehalten wird, wobei die Rührgeschwindigkeit derart eingestellt wird, daß sie ausreichend ist, das während der Reaktion aus dem Reaktionsmedium in Form eines kristallinen Pulvers ausfallende Hexakis-(hydroxymethyl)-melamin in dem Reaktionsmedium homogen verteilt zu halten;

(c) weitere Umsetzung des Reaktionsgemisches, das in (b) erhalten wurde, drei Stunden lang unter Rühren, bis zur Beendigung der Hydroxymethylierungsreaktion, worauf die Temperatur des Reaktionsgemisches und der mit ihm in Kontakt befindlichen mechanischen Teile auf einen Wert von etwa 45°C gesenkt wird, während der pH-Wert bei etwa 9 gehalten wird;

(d) Abtrennung des so erhaltenen Hexakis-(hydroxymethyl)-melamins vom übrigen Reaktionsmedium durch Filtration, wobei das Reaktionsmedium auf einer Temperatur von etwa 45°C und bein einem pH-Wert von etwa 9 gehalten wird, sich das Hexakis-(hydroxymethyl)-melamin in Form eines kristallinen Pulvers befindet und dieses leicht filtrierbar und in einem Zustand ist, der es für die nachfolgende Veretherung mit Methanol zu Hexakis-(methoxymethyl)-melamin geeignet macht.

2. Verfahren nach Punkt 1, gekennzeichnet dadurch, daß die Abtrennung durch Filtration in (d) dadurch ausgeführt wird, daß das übrige Reaktionsmedium aus dem Reaktionsgefäß entfernt wird und daß die Veretherung im gleichen Reaktionsgefäß durchgeführt wird.

3. Nach dem Verfahren nach Punkt 1 oder 2 hergestelltes Hexakis-(methoxymethyl)-melamin, gekennzeichnet dadurch, daß es in einem beliebigen Verhältnis in Wasser vollständig löslich ist und daß es eine Viskosität von weniger als 2500 cP (bei 20°C und mit einem Feststoffgehalt von 95%) aufweist und daß das besagte Hexakis-(methoxymethyl)-melamin einen mittleren Methylierungsgrad von nicht weniger als 5,8 aufweist.

**Revendications**

1. Procédé pour la préparation d'hexacis-(méthoxyméthyl)mélamine hydrosoluble, comprenant la réaction de méthylolation de la mélamine avec la formaldéhyde en solution aqueuse pour obtenir l'hexacis(hydroxyméthyl)-mélamine, la séparation de celle-ci du milieu de réaction résiduel et le réaction d'éthérification au méthanol de dite hexacis(hydroxyméthyl)-mélamine, caractérisé du fait que dite réaction de méthylolation comprend:

(a) préparation d'un mélange de mélanine en solution aqueuse de formaldéhyde dont le pH soit réglé à une valeur de 9 environ, avec un rapport molaire mélamine-formaldéhyde d'environ 1:11, et un contenu d'eau d'environ du 44% en poids, basé sur le poids total de dit mélange;

(b) réaction sous agitation du mélange susdit pour une heure environ à une température de 70°C à 75°C et à un pH de 9 à peu près, tout en maintenant la masse de réaction sous agitation à une rapidité d'agitation suffisante à peine pour que l'hexacis(hydroxyméthyl)mélamine, qui précipite dans le milieu de réaction, se présente sous forme de poudre cristalline suspendue fans le milieu susdit d'une façon substantiellement homogène;

(c) réaction ultérieure sous agitation pour trois heures environs de la masse de réaction obtenue de la manière dont au point (b) jusqu'à ce que la réaction de méthylolation soit complétée, en réduisant et en maintenant la température de dite masse de réaction et des parties mécaniques à contact avec elle à une valeur de 45°C environ et le pH à 9 environ.

(d) séparation par filtration, du milieu de réaction résiduel, de l'hexacis(hydroxyméthyl)-mélamine obtenue de la manière susdite, en maintenant dit milieu de réaction résiduel à une température de 45°C environ et à un pH de 9 environ, dite hexacis(hydroxyméthyl)mélamine se présentant sous forme de poudre cristalline, facile à filtrer et prête à être successivement éthérifiée au methanol en hexacis(méthoxy-méthyl)mélamine.

2. Procédé dont à la revendication 1, caractérisé du fait que la séparation par filtration dont au point (d) est obtenue par extraction de dit milieu de réaction résiduel du réacteur dé synthèse, et que dite réaction d'éthérification est effectuée dans ce même réacteur de synthèse.

3. Hexacis(méthoxyméthyl)mélamine préparée selon le procédé dont aux revendications 1 ou 2, caractérisée du fait d'être entièrement hydrosoluble dans n'importe quel rapport, et d'avoir une viscosité inférieure à 2500 cps (à 20°C et par un contenu solide du 95%), dite hexacis(méthoxy-méthyl)mélamine ayant un degré moyen de méthylation qui n'est pas inférieur à 5,8.